# EUROPEAN PATENT SPECIFICATION

(11) **EP 0 347 243 B1**
(45) Date of publication and mention of the grant of the patent: **13.01.1993**
(21) Application number: 89306121.8
(22) Date of filing: 16.06.1989
(51) Int. Cl.: A61K 31/557, A61K 31/34, A61K 31/40, A61K 49/00

(54) **Prostaglandin analogues for use in medicine**
Prostaglandinanaloge zur Verwendung in der Medizin
Analogues de prostaglandines pour l'utilisation médicale

(30) Priority: 17.06.1988 GB 8814438; 14.10.1988 GB 8824187
(43) Date of publication of application: 20.12.1989
(73) Proprietor: THE WELLCOME FOUNDATION LIMITED, London NW1 2BP (GB)
(72) Inventor: Tadepalli, Anjaneyulu Seetharam, Durham North Carolina 27705 (US); Long, Walker Anderson, Chapel Hill North Carolina 27514 (US); Crow, James Walker, Raleigh North Carolina 27609 (US); Klein, Kenneth Bruce, Chapel Hill North Carolina 27514 (US); Whittle, Brendan Joseph Richard, Beckenham Kent (GB)
(74) Representative: Garrett, Michael

(56) References cited:
- EP-A- 0 005 768
- EP-A- 0 034 778
- EP-A- 0 217 419
- EP-A- 0 229 844
- US-A- 4 306 075
- US-A- 4 499 085
- CIRCULATION, vol. 66, 1982, American Heart Association, Inc. L.J.RUBIN et al. "Prostacyc- lin-induced Acute Pulmonary Vasodilation in Primary Pulmonary Hypertension" pages 334-338
- PROGRESS IN MEDICINAL CHEMISTRY, vol. 21, 1984, Elsevier Science Publishers B.J.R. WHITTLE et al. "6 Antithrombotic Assessment and Clinical Potential of Prostacyclin Analogues" pages 238-279
- AUSTRIA-CODEX FACHINFORMATION 1987/88, Österreichische Apotheker-Verlagsgesell- schaft M.B.H., Wien, 1987 pages 779-780
- CIRCULATION, vol. 72, 1985, American Heart Association B.J.R.WHITTLE et al. "Platelet actions of stable carbocyclic analogues of prostacyclin" pages 1219-1225

## Description

The present invention is concerned with the use of prostaglandins in the manufacture of medicaments for the prophylaxis or treatment of pulmonary hypertension.

All blood is driven through the lungs via the pulmonary circulation in order, among other things, to replenish the oxygen which it dispenses in its passage around the rest of the body via the systemic circulation. The flow through both circulations is in normal circumstances equal, but the resistance offered to it in the pulmonary circulation is generally much less than that of the systemic circulation. When the resistance to pulmonary blood flow increases, the pressure in the circulation is greater for any particular flow. This is referred to as pulmonary hypertension. Generally, pulmonary hypertension is defined through observations of pressures above the normal range pertaining in the majority of people residing at the same altitude and engaged in similar activities.

Most often pulmonary hypertension is a manifestation of an obvious or explicable increase in resistance, such as obstruction to blood flow by pulmonary emboli, malfunction of the heart's valves or muscle in handling blood after its passage through the lungs, diminution in pulmonary vessel calibre as a reflex response to hypoventilation and low oxygenation, or a mismatch of vascular capacity and essential blood flow, such as shunting of blood in congenital abnormalities or surgical removal of lung tissue. Such pulmonary hypertension is referred to as secondary hypertension.

There remain some cases of pulmonary hypertension where the cause of the increased resistance is as yet inexplicable. These are described as cases of primary pulmonary hypertension (PPH) and are diagnosed by and after exclusion of the causes of secondary pulmonary hypertension. Despite the possibility of a varied aetiology, cases of primary pulmonary hyptertension tend to comprise a recognisable entity. Approximately 65% are female and young adults are most commonly afflicted, though it has occurred in children and patients over 50. Life expectancy from the time of diagnosis is short, about 3 to 5 years, though occasional reports of spontaneous remission and longer survival are to be expected given the nature of the diagnostic process. Generally, however, progress is inexorable via syncope and right heart failure and death is quite often sudden. Until now, no successful treatment was known.

Circulation 66, 334 (1982) describes a study to evaluate the effects of prostacyclin on pulmonary vascular tone in PPH wherein it was demonstrated that prostacyclin can increase cardiac output and reduce pulmonary arterial pressure and resistance in patients having said condition.

Progress in Medicinal Chemistry 21, 237 (1984) is a review of the clinical potential of prostacyclin analogues wherein it is indicated that 9-deoxy-9α-6-nitrilo-PGF₁, like prostacyclin, is a pulmonary vasodilator in the lamb, under both hypoxic and normoxic conditions, and reduces the pulmonary-systemic resistance ratio during hypoxia. It is stated to be some 5 - 17-fold less active than prostacyclin.

U.S. Patent 4,306,075 describes novel benzindene prostaglandins which produce various pharmacological responses, such as inhibition of platelet aggregation, reduction of gastric secretion and bronchodilation. It is indicated that these compounds have useful application as anti-thrombotic agents, anti-ulcer agents and anti-asthma agents. Non-benzindene prostaglandins having similar properties have also been described (Progress in Medicinal Chemistry, 21, 237 (1984); Circulation, 72, 1219 (1985)). We are not aware of any disclosure to date that benzindene or non-benzindene prostaglandins other than prostacyclin, 9-deoxy-9x-6-nitrilo-PGF₁ and PGE₁ may be suitable for use in the prophylaxis, treatment, or diagnosis of pulmonary hypertension.

We have now identified a class of prostaglandins comprising known benzindenes and non-benzindenes which have unexpectedly been found suitable for use in the prophylaxis, treatment and diagnosis of pulmonary hypertension. The compounds of the invention may also be used in the prophylaxis and treatment of Raynaud's disease.

The term "pulmonary hypertension" is used herein to include both primary and secondary pulmonary hypertension as ordinarily understood by clinicians (vide supra).

PPH patients having active pulmonary vasoconstriction are considered suitable candidates for long-term oral vasodilator therapy (R J Lambert et al, Chest 89, 459S (1986)). The ability of the compounds of the invention to reduce pulmonary vascular resistance in such patients provides a useful diagnostic aid for identifying suitable candidates for long-term vasodilator therapy.

The present invention, therefore, lies in the use of a compound of formula (I) 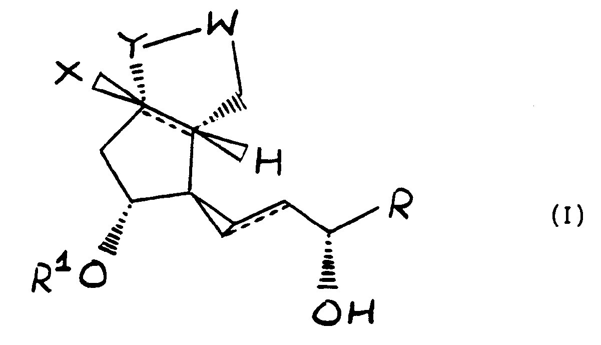
wherein
-W- is selected from 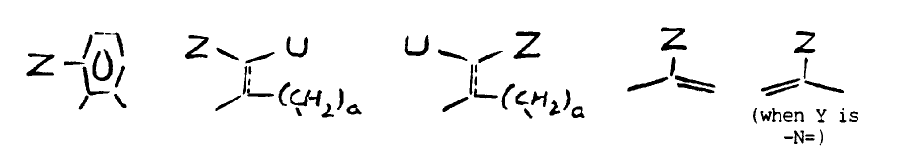
where a is 0 or 1, U is hydrogen or halogen and Z is -V(CH₂)_{b}CO₂H where b is an integer of from 1 to 3 and V is oxygen or methylene;
X is hydrogen, methyl, halogen, cyano, or -C≡CH;
Y is oxygen, methylene, -N=, or -N(Ar)- where Ar a phenyl group optionally meta- substituted by a melthylthio group;
R is -(CH₂)₅R² where R² is hydrogen or methyl, or R is cyclohexyl, or R is -CH(CH₃)CH₂C≡CCH₃;
R¹ is hydrogen or methyl; and
the dotted lines represent independently optional double bonds;
provided said compound of formula (I) is not 6,9-epoxy-11,15-dihydroxyprosta-5,13-dienoic acid or 9-deoxy-9α-6-nitrilo-PGF₁;
and physiologically acceptable salts and acid derivatives thereof,
in the manufacture of a medicament for the prophylaxis or treatment of pulmonary hypertension.

The term "acid derivative" is used herein to describe C₁₋₄ alkyl esters and amides, including amides wherein the nitrogen is optionally substituted by one or two C₁₋₄ alkyl groups.

The invention also includes the use of bioprecursors or "pro-drugs" of the above-defined compounds, that is, compounds which are converted in vivo to compounds of formula (I) or physiologically active derivatives thereof.

A further aspect of the present invention provides for the use of a compound of formula (I), or a physiologically acceptable salt or acid derivative thereof, in the manufacture of a diagnostic aid for identifying PPH patients having active pulmonary vasoconstriction. Medicaments and diagnostic aids obtained by the use of the invention which may be administered when primary or secondary pulmonary hypertension is indicated are also within the scope of the invention.

Preferred compounds of formula (I) having particularly desirable pulmonary anti-hypertensive properties include those wherein
-W- is 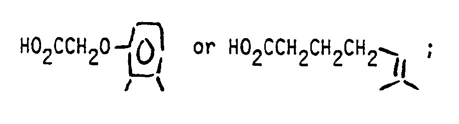
Y is methylene;
R is -(CH₂)₅R² is hydrogen; and
R¹ is hydrogen;
and physiologically acceptable salts and acid derivatives thereof.

Particularly preferred compounds of formula (I) having exceptional pulmonary anti-hypertensive properties are 9-deoxy-2′,9α-methano-3-oxa-4, 5,6-trinor-3,7-(1′,3′-interphenylene)-13,14-dihydroprostaglandin F₁ (A) and (5Z,9S)-9-methyl-6a-carbaprostaglandin I₂ (B) which have the following structures: 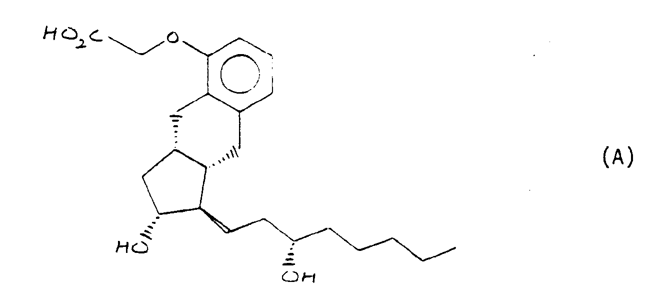 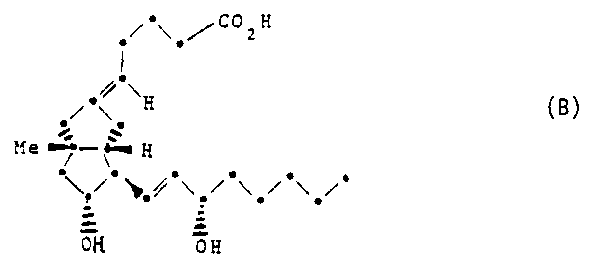
and physiologically acceptable salts and acid derivatives of both thereof.

Other compounds of the invention which show pulmonary anti-hypertensive activity include :
9-Deoxy-2′,9-methano-3-oxa-4,5,6-trinor-3,7-(1′,3′-interphenylene)prostaglandin F₁
9-Deoxy-2′,9α-methano-3-oxa-4,5,6-trinor-3,7-(1′,3′-interphenylene)-15-cyclohexylprostaglandin F₁
9-Deoxy-2′,9-methano-3-oxa-4,5,6-trinor-3,7-(1′,3′-interphenylene)-20-methylprostaglandin F₁
Carbacyclin (6a-carba-PGI₂)
5,6-Dihydroprostacyclin
9-Deoxy-5R,9α-epoxyprostaglandin F_{1α}
(6R)-5-Oxa-6a-carbaprostaglandin I₁
(5E)-5-Fluoro-6a-carbaprostaglandn I₂
9-Deoxy-5,9-methano-4,5-didehydroprostaglandin F_{1α}
(5Z,9R)-9-Chloro-6a-carbaprostaglandin I₂
(5Z,9R)-9-Cyano-6a-carbaprostaglandin I₂
(15S,16RS)-9-Deoxy-2′9α-methano-16-methyl-3-oxa-18,18,19,19-tetradehydro-4,5,6-trinor-3,7-(1′,3′-interphenylene)prostaglandin F₁
(5Z,9R)-9-Ethynyl-6a-carbaprostaglandin I₂
(5Z,9R,16RS)-9-Ethynyl-16-methyl-18,18,19,19-tetradehydro-6a-carbaprostaglandin I₂
(11ξ)-6a-(3-Methylthiophenyl)-6,7,8,9-tetradehydro-6a-azaprostaglandin I₁ methyl ester 11-methyl ether

The present invention extends to non-physiologically acceptable salts of the compounds of formula (I) which may be used in the preparation of the pharmacologically-active compounds of the invention. The physiologically acceptable salts of compounds of formula (I) include salts derived from organic and inorganic acids as well as from bases. Suitable salts derived from acids include, for example, the acetate, adipate, alginate, aspartate, benzoate, benzenesulphonate, bisulphate, butyrate, citrate, camphorate, camphorsulphonate, cyclopentanepropionate, digluconate, dodecylsulphate, ethanesulphonate, fumarate, glucoheptanoate, glycerophosphate, hemisulphate, heptanoate, hexanoate, hydrochloride, hydrobromide, hydroiodide, 2-hydroxyethanesulphonate, lactate, maleate, methanesulphonate, 2-naphthalenesulphonate, nicotinate, oxalate, palmoate, pectinate, persulphate, 3-phenylpropionate, picrate, pivalate, propionate, succinate, tartrate, thiocyanate, tosylate, and undecanoate.

Base salts include ammonium salts, alkali metal salts such as those of sodium and potassium, alkaline earth metal salts such as those of calcium and magnesium, salts with organic bases such as dicyclohexylamine and N-methyl-D-glucamine, and salts with amino acids such as arginine and lysine.

Quaternary ammonium salts can be formed, for example, by reaction with lower alkyl halides, such as methyl, ethyl, propyl, and butyl chlorides, bromides, and iodides, with dialkyl sulphates, with long chain halides, such as decyl, lauryl, myristyl, and stearyl chlorides, bromides, and iodides, and with aralkyl halides, such as benzyl and phenethyl bromides.

The amount of a compound of formula (I), or a physiologically acceptable salt or acid derivative thereof, which is required in a medication or diagnostic aid according to the invention to achieve the desired effect will depend on a number of factors, in particular the specific application, the nature of the particular compound used, the mode of administration, and the condition of the patient. In general, a daily dose for the prophylaxis or treatment of pulmonary hypertension is expected to lie in the range 25 µg to 250 mg, typically from 0.5 µg to 2.5 mgₓ per day per kilogram bodyweight. For example, an intravenous dose may be in the range 0.5 µg to 1.5 mg/kg/day, which may conveniently be administered as an infusion of from 0.5 ng to 1.0 µg per kilogram per minute. Infusion fluids suitable for this purpose may contain, for example, from 10 ng to 10 µg per millilitre. Ampoules for injection may contain, for example, from 0.1 µg to 1.0 mg and orally administrable unit dose formulations, such as tablets or capsules, may contain, for example, from 0.1 to 100 mg, typically from 1 to 50 mg. For diagnostic purposes, a single unit dose formulation may be administered. In the case of physiologically acceptable salts, the weights indicated above refer to the weight of the active compound ion, that is, the ion derived from the compound of formula (I).

In the manufacture of a medicament or diagnostic aid according to the invention, hereinafter referred to as a "formulation", the compounds of formula (I) and their physiologically acceptable salts and acid derivatives are typically admixed with, inter alia, one or more carriers and/or excipients. The latter must, of course, be acceptable in the sense of being compatible with any other ingredients in the formulation and must not be deleterious to the patient. The carrier may be a solid or a liquid, or both, and is preferably formulated with the compound as a unit-dose formulation, for example, a tablet, which may contain from 0.05% to 95% by weight of the active compound. One or more compounds of formula (I) and/or their physiologically acceptable salts or acid derivatives may be incorporated in the formulations of the invention, which may be prepared by any of the well known techniques of pharmacy consisting essentially of admixing the components.

In addition to compounds of formula (I), other pharmacologically active substances may be present in the medicaments of the present invention. for example, the compounds of the invention may be present in combination with tissue plasminogen activator, a substance known to dissolve the fibrin network of blood clots which has found utility in the treatment of thrombotic disorders (see, for example, The New England Journal of Medicine, 312(14), 932, (1985)).

The formulations of the invention include those suitable for oral, rectal, topical, buccal (e.g. sub-lingual), parenteral (e.g. subcutaneous, intramuscular, intradermal, or intravenous) and transdermal administration, although the most suitable route in any given case will depend on the nature and severity of the condition being treated and on the nature of the particular compound of formula (I), or the physiologically acceptable salt or acid derivative thereof, which is being used.

Formulations suitable for oral administration may be presented in discrete units, such as capsules, cachets, lozenges, or tablets, each containing a predetermined amount of a compound of formula (I) or a physiologically acceptable salt or acid derivative thereof; as a powder or granules; as a solution or a suspension in an aqueous or non-aqueous liquid; or as an oil-in-water or water-in-oil emulsion. Such formulations may be prepared by any suitable method of pharmacy which includes the step of bringing into association the active compound and a suitable carrier (which may contain one or more accessory ingredients). In general, the formulations of the invention are prepared by uniformly and intimately admixing the active compound with a liquid or finely divided solid carrier, or both, and then, if necessary, shaping the resulting mixture. For example, a tablet may be prepared by compressing or moulding a powder or granules containing the active compound, optionally with one or more accessory ingredients. Compressed tablets may be prepared by compressing, in a suitable machine, the compound in a free-flowing form, such as a powder or granules optionally mixed with a binder, lubricant, inert diluent, and/or surface active/dispersing agent(s). Moulded tablets may be made by moulding, in a suitable machine, the powdered compound moistened with an inert liquid binder.

Formulations suitable for buccal (sub-lingual) administration include lozenges comprising a compound of formula (I), or a physiologically acceptable salt or acid derivative thereof, in a flavoured base, usually sucrose and acacia or tragacanth; and pastilles comprising the compound in an inert base such as gelatin and glycerin or sucrose and acacia.

Formulations of the present invention suitable for parenteral administration conveniently comprise sterile aqueous preparations of a compound of formula (I), or a physiologically acceptable salt or acid derivative thereof, which preparations are preferably isotonic with the blood of the intended recipient. These preparations are preferably administered intravenously, although administration may also be effected by means of subcutaneous, intramuscular, or intradermal injection. Such preparations may conveniently be prepared by admixing the compound with water or a glycine buffer and rendering the resulting solution sterile and isotonic with the blood. Injectable formulations according to the invention will generally contain from 0.1 to 5% w/v of active compound and be administered at a rate of 0.1 ml/min/kg.

Formulations suitable for rectal administration are preferably presented as unit dose suppositories. These may be prepared by admixing a compound of formula (I), or a physiologically acceptable salt or acid derivative thereof, with one or more conventional solid carriers, for example, cocoa butter, and then shaping the resulting mixture.

Formulations suitable for topical application to the skin preferably take the form of an ointment, cream, lotion, paste, gel, spray, aerosol, or oil. Carriers which may be used include vaseline, lanoline, polyethylene glycols, alcohols, and combinations of two or more thereof. The active compound is generally present at a concentration of from 0.1 to 15% w/w, for example, from 0.5 to 2% w/w.

Formulations for transdermal administration may be delivered by iontophoresis (see, for example, Pharmaceutical Research 3(6), 318, (1986)) and typically take the form of an optionally buffered aqueous solution of a compound of formula (I) or of a salt or acid derivative thereof. Suitable formulations comprise citrate or bis/tris buffer (pH 6) or ethanol/water and contain from 0.1 to 0.2M active ingredient.

The compounds of the present invention are conveniently prepared by methods the same as or analogous to those described in U.S. Patent 4,306,075.

For a better understanding of the invention, the following Examples are given by way of illustration.

### EXAMPLES

The effects of 9-deoxy-2′,9-methano-3-oxa-4,5,6-trinor-3,7(1′,3′-interphenylene)-13,14-dihydro-prostaglandin F₁ (Example 1) and (5Z,9S)-9-methyl-6a-carbaprostaglandin I₂ (Example 2) were monitored in experimental pulmonary hypertension models. In both Examples, the model used was an open chest preparation of an anaesthesised cat (anaesthetic: chloralose and urethane).

### Example 1

A series of glycine buffer solutions (pH 10.5) of the test compound were successively administered to each of four animals by i.v. infusion at doses equivalent to 100ng, 300ng, 1µg, and 3µg/kg/min. Each solution was infused over a period of 20 minutes, hypoxia being induced in the animal during the last 5 minutes of infusion by ventilating with 10% oxygen in nitrogen. A 15-minutes 'recovery' period was observed between successive infusions. Following surgery, the animal was allowed to stabilize for 30 minutes, after which two 5-minute hypoxic challenges were given 15 minutes apart which were averaged to obtain the control hypoxic responses. 15 minutes after the second control hypoxic challenge, the animal started to receive the test compound. The averaged control hypoxic responses were compared with those obtained during infusion of the test compound.

The following parameters were monitored during the course of each experiment: systemic arterial pressure (MAP), pulmonary arterial (PAP) and venous (PVP) pressures, and cardiac output (CO, aortic blood flow). From the values obtained, the systemic vascular resistance (MAP/CI where CI = CO/body weight in kg) and the pulmonary vascular resistance (PAP/CI) were calculated.

The test compound was found to reduce hypoxia-induced increase in pulmonary arterial pressure and pulmonary vascular resistance in a dose-related manner without appreciably affecting cardiac output or heart rate. At higher doses, the test compound reduced systemic arterial pressure and systemic vascular resistance. Thus hypoxia-induced pulmonary vasoconstriction could be reduced without disturbing the systemic haemodynamics by suitably adjusting the dose. The hypoxia-induced vasoconstriction did not return to its control value within 15 minutes of terminating the final infusion indicating a relatively long duration of action for the compound.

### Example 2

A series of glycine buffer solutions (pH 10.5) of the test compound were successfully administered to each of five animals by i.v. infusion at doses equivalent to 300ng, 1µg, 3µg, 10µg and 30µg/kg/min. Each solution was infused over a period of 20 minutes, hypoxia being induced in the animal during the last 5 minutes of infusion by ventilating with 10% oxygen in nitrogen. A 15-minute 'recovery' period was observed between successive infusions. Following surgery, the animal was allowed to stabilize for 30 minutes, after which two 5-minute hypoxic challenges were given 15 minutes apart which were averaged to obtain the control hypoxic responses. 15 minutes after the second control hypoxic challenge, the animal started to receive the test compound. The averaged control hypoxic responses were compared with those obtained during infusion of the test compound.

The following parameters were monitored during the course of each experiment: systemic arterial pressure (MAP), pulmonary arterial (PAP) and venous (PVP) pressures, and cardiac output (CO, aortic blood flow). From the values obtained, the systemic vascular resitance (MAP/CI) and the pulmonary vascular resistance (PAP/CI) were calculated.

The test compound was found to reduce hypoxia-induced increase in pulmonary arterial pressure and pulmonary vascular resistance in a dose-related manner without appreciably affecting cardiac output or heart rate. At higher doses, the test compound reduced systemic arterial pressure and systemic vascular resistance. Thus hypoxia-induced pulmonary vasoconstriction could be reduced without disturbing the systemic haemodynamics by suitably adjusting the dose. The hypoxia-induced vasoconstriction did not return to its control value within 15 minutes of terminating the final infusion indicating a relatively long duration of action for the compound.

## Claims

1. Use of a compound of formula (I) 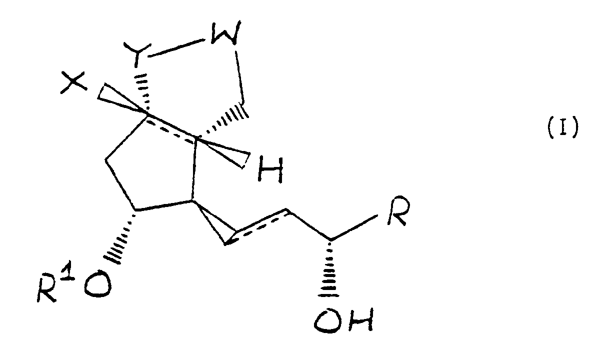 wherein
-W- is selected from 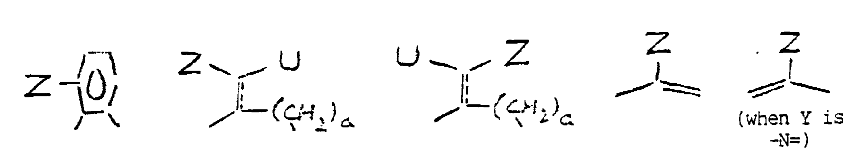 where a is 0 or 1, U is hydrogen or halogen and Z is -V(CH₂)_{b}-CO₂H where b is an integer of from 1 to 3 and V is oxygen or methylene;
X is hydrogen, methyl, halogen, cyano, or -C≡CH;
Y is oxygen, methylene, -N=, or -N(Ar)- where Ar is a phenyl group optionally meta-substituted by a methylthio group;
R is -(CH₂)₅R² where R² is hydrogen or methyl, or R is cyclohexyl, or R is -CH(CH₃)CH₂C≡CCH₃;
R¹ is hydrogen or methyl; and
the dotted lines represent independently optional double bonds;
provided said compound of formula (I) is not 6,9-epoxy-11,15-dihydroxyprosta-5,13-dienoic acid or 9-deoxy-9α-6-nitrilo-PGF₁;
and physiologically acceptable salts and acid derivatives thereof,
in the manufacture of a medicament for the prophylaxis or treatment of pulmonary hypertension.

2. Use of a compound of formula (I) 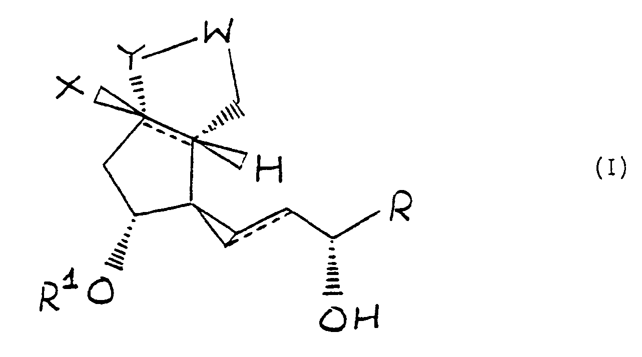 wherein
-W- is selected from 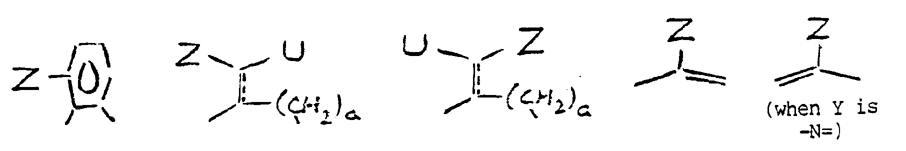 where a is 0 or 1, U is hydrogen or halogen and Z is -V(CH₂)_{b}-CO₂H where b is an integer of from 1 to 3 and V is oxygen or methylene;
X is hydrogen, methyl, halogen, cyano, or -C≡CH;
Y is oxygen, methylene, -N=, or -N(Ar)- where Ar is a phenyl group optionally meta-substituted by a methylthio group;
R is -(CH₂)₅R² where R² is hydrogen or methyl, or R is cyclohexyl, or R is -CH(CH₃)CH₂C≡CCH₃;
R¹ is hydrogen or methyl; and
the dotted lines represent independently optional double bonds;
provided said compound of formula (I) is not 6,9-epoxy-11,15-dihydroxyprosta-5,13-dienoic acid or 9-deoxy-9α-6-nitrilo-PGF₁;
and physiologically acceptable salts and acid derivatives thereof,
in the manufacture of a diagnostic aid for identifying PPH patients having active pulmonary vasoconstriction.

3. Use according to claim 1 or 2, wherein the compound of formula (I) is as shown in claim 1 wherein
-W- is 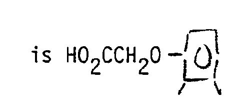 or 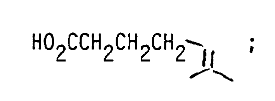 Y is methylene;
R is -(CH₂)₄CH₃; and
R¹ is hydrogen.

4. Use according to claim 3, wherein the compound of formula (I) is 9-deoxy-2′,9-methano-3-oxa-4,5,6-trinor-3,7-(1′,3′-interphenylene)-13,14-dihydroprostaglandin F₁, (5Z,9S)-9-methyl-6a-carbaprostaglandin I₂, or a physiologically acceptable salt or acid derivative of either thereof.

## Patentansprüche

1. Verwendung einer Verbindung der Formel (I) 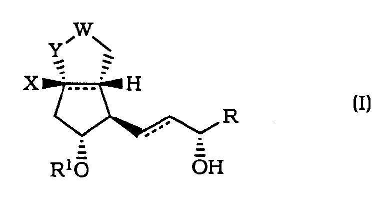 in der
-W- ausgewählt ist aus 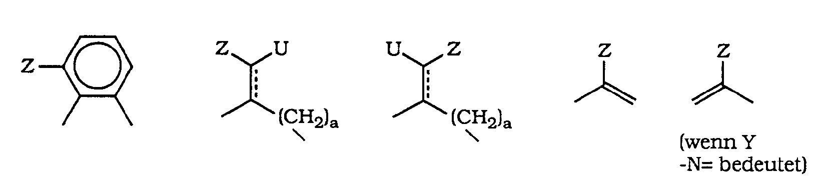 worin a 0 oder 1, U Wasserstoff oder Halogen und Z-V(CH₂)_{b}- CO₂H darstellen, worin b eine ganze Zahl mit einem Wert von 1 bis 3 und V Sauerstoff oder Methylen darstellen;
X Wasserstoff, Methyl, Halogen, Cyano oder -C≡CH;
Y Sauerstoff, Methylen, -N= oder -N(Ar)-, worin Ar eine gegebenenfalls in der meta-Stellung durch eine Methylthiogruppe substituierte Phenylgruppe darstellt;
R -(CH₂)₅R², worin R² Wasserstoff oder Methyl oder R Cyclohexyl oder R -CH(CH₃)CH₂C≡CCH₃ darstellt;
R¹ Wasserstoff oder Methyl und
die gestrichelten Linien unabhängig voneinander gegenbenenfalls vorhandene Doppelbindungen bedeuten, mit der Maßgabe, daß die Verbindung der Formel (I) nicht 6,9-Epoxy- 11, 15-dihydroxy-prosta-5, 13-diensäure oder 9-Deoxy-9α-6-nitrilo-PGF₁ ist;
und deren physiologisch annehmbare Salze und Säurederivate davon, bei der Herstellung eines Arzneimittels zur Prophylaxe oder Behandlung von Lungenhypertension.

2. Verwendung einer Verbindung der Formel (I) 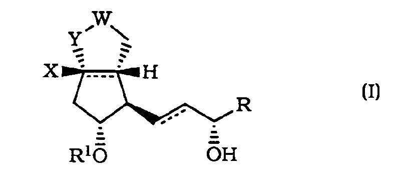 in der
-W- ausgewählt ist aus 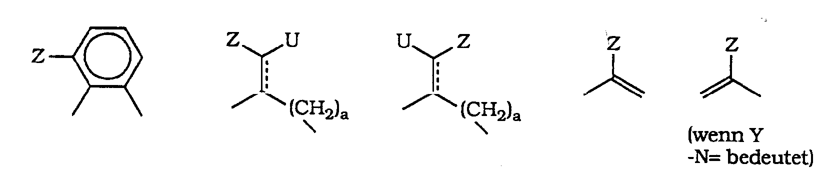 worin a 0 oder 1, U Wasserstoff oder Halogen und Z -V(CH₂)_{b}-CO₂H darstellen, in der b eine ganze Zahl mit einem Wert von 1 bis 3 und V Sauerstoff oder Methylen darstellt;
X Wasserstoff. Methyl, Halogen, Cyano oder -C≡CH;
Y Sauerstoff, Methylen, -N= oder -N(Ar)-, worin Ar eine Phenylgruppe, die gegebenenfalls in der meta-Stellung durch eine Methylthiogruppe substituiert ist, darstellt;
R -(CH₂)₅R², in der R² Wasserstoff oder Methyl darstellt, oder R Cyclohexyl oder R -CH(CH₃)CH₂C≡CCH₃;
R¹ Wasserstoff oder Methyl und
die gestrichelten Linien unabhängig voneinander gegebenenfalls vorhandene Doppelbindungen bedeuten; mit der Maßgabe, daß die Verbindung der Formel (I) nicht 6,9-Epoxy-11,15-dihydroxy-prosta-5,13-diensäure oder 9-Deoxy-9α-6-nitrilo-PGF₁ ist;
und deren physiologisch annehmbare Salze und Säurederivate zur Herstellung eines Diagnosehilfsmittels zur Identifizierung von PPH-Patienten (an primärer Lungenhypertension leidenden Patienten), die eine aktive Lungengefäßverengung aufweisen.

3. Verwendung nach Anspruch 1 oder 2, worin die Verbindung der Formel (I) wie in Anspruch 1 dargestellt ist, worin
-W- 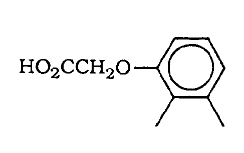 oder 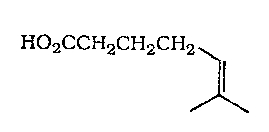 Y Methylen;
R -(CH₂)₄CH₃; und
R¹ Wasserstoff bedeuten.

4. Verwendung nach Anspruch 3, worin die Verbindung der Formel (I) 9-Deoxy-2′,9-methano-3-oxa-4,5,6-trinor-3,7-(1′,3′-interphenylen)- 13, 14-dihydroprostaglandin-F₁, (5Z,9S) -9-Methyl-6a-carbaprostaglandin-I₂ oder ein physiologisch annehmbares Salz oder Säurederivat einer dieser Verbindungen ist.

## Revendications

1. Utilisation d'un composé de formule (I) : 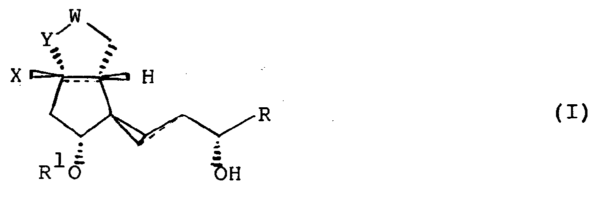 où
-W- est choisi parmi : 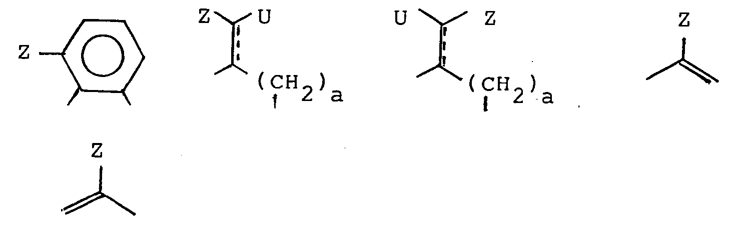 (lorsque Y est -N=)
où a est 0 ou 1, U est hydrogène ou halogène et Z est -V(CH₂)_{b}CO₂H, où b est un entier de 1 à 3 et V est oxygène ou méthylène;
X est hydrogène, méthyle, halogène, cyano ou -C≡CH;
Y est oxygène, méthylène, -N= ou -N(Ar)-, où Ar est un radical phényle facultativement méta-substitué par un radical méthylthio;
R est -(CH₂)₅R², où R² est hydrogène ou méthyle, ou R est cyclohexyle, ou R est -CH(CH₃)CH₂C≡CCH₃;
R¹ est hydrogène ou méthyle, et
les lignes en pointillé représentent des doubles liaisons indépendamment facultatives;
avec la restriction que le composé de formule (I) n'est pas l'acide 6,9-époxy-11,15-dihydroxy-prosta-5,13-diénoïque ni la 9-désoxy-9α-6-nitrilo-PGF₁;
et de ses sels et dérivés d'acide physiologiquement acceptables dans la fabrication d'un médicament pour la prophylaxie ou le traitement de l'hypertension pulmonaire.

2. Utilisation d'un composé de formule (I) : 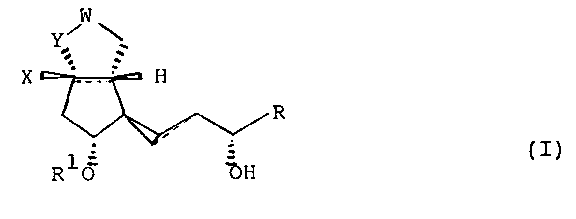 où
-W- est choisi parmi : 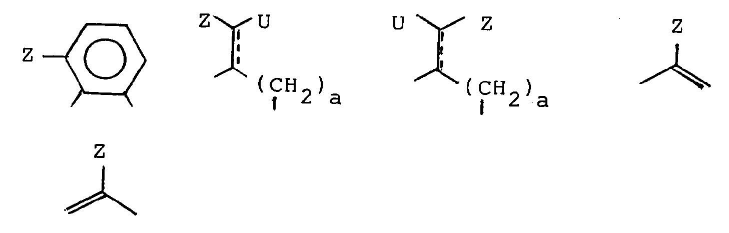 (lorsque Y est -N=)
où a est 0 ou 1, U est hydrogène ou halogène et Z est -V(CH₂)_{b}CO₂H, où b est un entier de 1 à 3 et V est oxygène ou méthylène;
X est hydrogène, méthyle, halogène, cyano ou -C≡CH;
Y est oxygène, méthylène, -N= ou -N(Ar)-, où Ar est un radical phényle facultativement méta-substitué par un radical méthylthio;
R est -(CH₂)₅R², où R² est hydrogène ou méthyle, ou R est cyclohexyle, ou R est -CH(CH₃)CH₂C≡CCH₃;
R¹ est hydrogène ou méthyle, et
les lignes en pointillé représentent des doubles liaisons indépendamment facultatives;
avec la restriction que le composé de formule (I) n'est pas l'acide 6,9-époxy-11,15-dihydroxy-prosta-5,13-diénoïque ni la 9-désoxy-9α-6-nitrilo-PGF₁;
et leurs sels et dérivés d'acide physiologiquement acceptables,
dans la fabrication d'un auxiliaire de diagnostic pour identifier les patients atteints d'hypertension pulmonaire primaire faisant de la vasoconstriction pulmonaire active.

3. Utilisation suivant la revendication 1 ou 2, dans laquelle le composé de formule (I) est tel qu'indiqué à la revendication 1, où :
-W- est 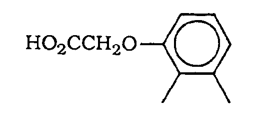 ou 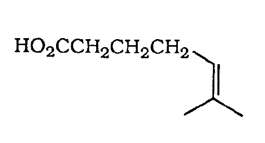 Y est méthylène;
R est -(CH₂)₄CH₃, et
R¹ est hydrogène.

4. Utilisation suivant la revendication 3, dans laquelle le composé de formule (I) est la 9-désoxy-2′,9-méthano-3-oxa-4,5,6-trinor-3,7-(1′,3′-interphénylène-13,14-dihydroprostaglandine F₁, la (5Z,9S)-9-méthyl-6a-carbaprostaglandine I₂ ou un sel ou dérivé d'acide physiologiquement acceptable de l'une ou l'autre.
